# EUROPEAN PATENT APPLICATION

(11) **EP 3 622 904 A1**
(43) Date of publication of application: **18.03.2020**
(21) Application number: 18194011.5
(22) Date of filing: 12.09.2018
(51) Int. Cl.: A61B 17/29, A61B 34/00, A61B 17/00

(54) **MINIMALLY INVASIVE SURGICAL MODULE**

(71) Applicant: Suzhou Kang Multi Robot Co Ltd, Suzhou City, Jiangsu (CN)
(72) Inventor: Du, Zhijiang, Suzhou City, Jiangsu Province (CN)
(74) Representative: Hellmich, Wolfgang

(57) **Abstract**

A minimally invasive surgical module for minimally invasive surgery which comprising a hand held portion 10, a power portion 20 which is fixedly connected with the hand held portion 10, a thin linking rod 30 which is fixedly connected with the power portion 20, an operative end portion 40 which is connected with the thin linking rod 30, and an operative system for controlling the operation of the power portion 20. Wherein, the hand held portion and the power portion are wirely connected with the operative system, the operation of the hand held portion triggers operations of encoders on the two joints provided on the instrument. The encoder collects the operation information of the joints and feeds back to the operative system , and the power portion receives signal from the operative system and drive the operative end portion to perform pitch, yaw, clamping motion.

## Description

### FIELD OF THE INVENTION

The present invention relates to surgical instruments, more particularly, relates to a minimally invasive surgical module.

### BACKGROUND OF THE INVENTION

A minimally-invasive procedure (MIP) typically involves use of arthroscopic (for joints and the spine) or laparoscopic devices and remote-control manipulation of instruments with indirect observation of the surgical field through an endoscope or large scale display panel, and is carried out through the skin or through a body cavity or anatomical opening. By use of a MIP, a patient may require only a band-aid on the incision, rather than multiple stitches or staples to close a large incision. This usually results in less infection, a quicker recovery time and shorter hospital stays.

Special medical equipment may be used, such as fiber optic cables, miniature video cameras and special surgical instruments handled via tubes inserted into the body through small openings in its surface to perform the surgery. However, the degree of freedom in the existing surgical instruments is limited, these surgical instruments typically do not of provide the pitch and yaw movement, therefore, they are more clumsy and can seriously affecting the quality and accuracy of the surgery. As such, an improvement in these surgical instruments are needed.

### SUMMARY OF THE INVENTION

The objective of the present invention is to provide a minimally invasive surgical module for minimally invasive surgery that is capable of yaw, pitch, clamping and axis rotation motion that increase the degree of freedom during an surgery in order to increase the quality and efficient of the surgery, and on the other hand, is compact in size.

The present invention provides the following solution:
a minimally invasive surgical module for minimally invasive surgery which comprising a hand held portion, a power portion which is fix connected with the hand held portion, a thin linking rod which is fix connected with the power portion, an operative end portion which is connected with the thin linking rod, and an operative system (not shown) for controlling the operation of the power portion;
the hand held portion which comprising a first linking rod that one end of the linking rod is fix connected with the power portion, a second linking rod which through a first joint, is routably connected with the first linking rod, a third linking rod which through a second joint, is rotablely connected with the second linking rod, a clamping unit which through a clamping cap, is fixed mounted on the top of the third linking rod, and encoders which are fixed mounted on the first joint and the second joint;
the clamping unit which comprising a base panel, the base panel is provided with a pair of symmetrically opposing planar linkage mechanisms, each of the linkage mechanisms which comprising a mover lever and a lower lever, one end of the mover lever is rotablely mounted on the base panel, and the other end of the mover lever is hinged to one end of the lower lever, and the other end of the lower lever is rotablely mounted on a protruding end of a potentiometer, the potentiometer is fix mounted on the base panel, a spring is provided in between the mover levers of the pair linkage mechanisms. The two ends of the spring are abutting against the two mover levers, and the side wall of the mover lever is provided with a clamping piece; and
the hand held portion and the power portion are both wire connected with the operative system. The operation of the hand held portion drives the encoder on the joints to operative, the encoder collects the operative information of the joints and then feeds back to the operative system, after the power portion receives signals from the operative system, the power portion drives the operative end portion to complete a yaw, pitch and clamping motion.

As a further improvement of the above described technical solution, the power portion which comprising an outer cover (not shown), the outer cover is provided with a yaw motor, a pitch motor and a clamping motor within. An output shaft end of the yaw motor is connected with an yaw motor active dial, an output shaft end of the pitch motor is connected with a pitch motor active dial, and an output shaft end of the clamping motor is connected with a clamping motor active dial; the yaw motor active dial is connected with a yaw motor passive dial, the pitch motor active dial is connected with a pitch motor passive dial, and the clamping motor active dial is connected with a clamping motor passive dial. The yaw motor passive dial, the pitch motor passive dial and the clamping motor passive dial are respectively connected with a metal wire turning apparatus.

As a further improvement of the above described technical solution, one end of the thin linking rod is fix arranged within the power portion and the other end of the thin linking rod extends out the power portion. Wherein, the thin linking rod is a hollow structure, and several fixing slots are provided within the hollow structure. The metal wire turning apparatus are fix arranged within the thin linking rod through the fixing slots.

As a further improvement of the above described technical solution, the metal wire turning apparatus are fix arranged within the thin linking rod through the fixing slots. The operative end portion connects with the thin linking rod at one end that is away from the power portion, and the operative end portion also connects with the metal wire turning apparatus.

As a further improvement of the above described technical solution, the side wall of the third linking rod is provided with a clutch button; the clutch button is arranged directly below the clamping unit and is wirely connected with operative system.

As a further improvement of the above described technical solution, the third linking rod is further provided with a bracelet, and the bracelet is arranged directly below the clutch button. Wherein, the bracelet and the clutch button are arranged on a same plane of the third linking rod.

As a further improvement of the above described technical solution, the bracelet which comprising a ring portion and connecting ends which are fix arranged at two ends of the ring portion. The connecting ends are fix mounted on the third linking rod. Wherein, the ring portion is made of elastic material and the connecting ends are made of hard material.

The effects of the present invention are that: the present invention provides the free motions of yaw, pitch, clamping and axis rotation, when a surgeon holds on the third linking rod, the surgeon can control the instrument to perform yaw, pitch and clamping motion through the operative end portion and the power potion. In addition, the turning of the surgeon's arm is reflected as an axis rotation of the operative end portion.

### BREIF DESCRIPTION OF THE DRAWINGS

Fig. 1 is a perspective structural view of the present invention;
Fig. 2 is a structural view of the bracelet in the present invention;
Fig. 3 is a structural view of the hand held portion in the present invention;
Fig. 4 is a structural view of the clamping unit in the present invention;
Fig. 5 is a structural view of the power portion in the present invention; and
Fig. 6 is a structural view of the metal wire turning apparatus in the present invention.

### DETAILED DECRIPTION OF THE INVENTION

Preferred embodiment: following is a preferred embodiment of the present invention, people skilled in art can easily understand the advantages and other technical effects of the present invention from the preferred embodiment. The "top" and "bottom" used in the preferred embodiment is defined as a general meaning of the term, for example, the direction of the gravitational force is downward, the opposite to that is upward, as such, the term "on the above" means "on the top" or "on the tip", and the term "at the below" means "on the bottom" or "at the bottom"; in addition, the terms of "first, second or third" used in the preferred embodiment is only a reference for easier explanation, they do not relate to the importance of the components nor a limitation to the preferred embodiment, and it should not be a limitation to the preferred embodiment.

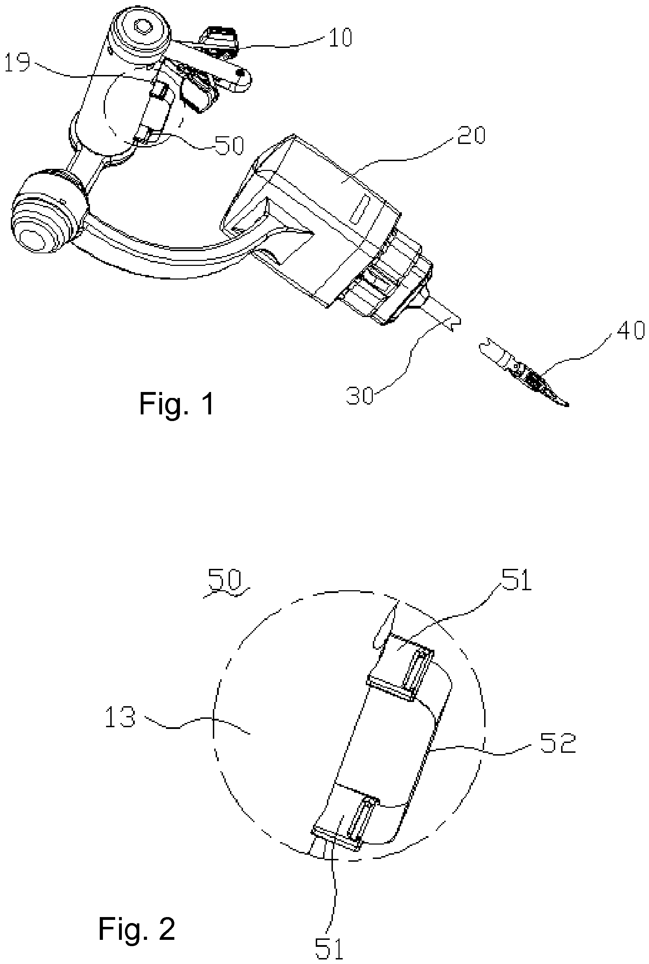

As shown in Fig. 1, A minimally invasive surgical module for minimally invasive surgery which comprising a hand held portion 10, a power portion 20 which is fix connected with the hand held portion 10, a thin linking rod 30 which is fix connected with the power portion 20, an operative end portion 40 which is connected with the thin linking rod 30, and an operative system (not shown) for controlling the operation of the power portion 20.

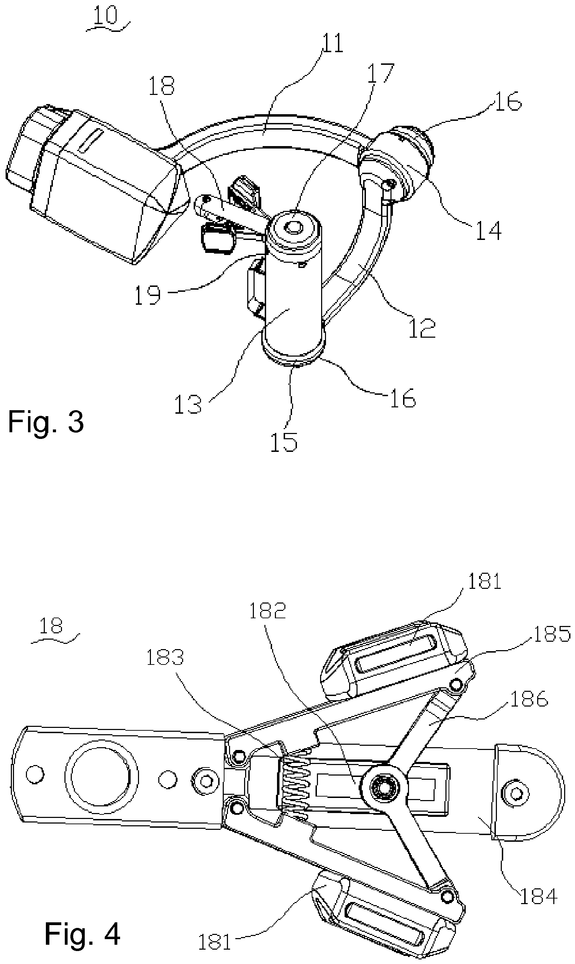

As shown in Fig. 3, the hand held portion 10 which comprising a first linking rod 11 that one end of the linking rod 11 is fix connected with the power portion 20, a second linking rod 12 which through a first joint 14, is rotablely connected with the first linking rod 11, a third linking rod 13 which through a second joint 15, is rotablely connected with the second linking rod 12, a clamping unit 18 which through a clamping cap 17, is fixed mounted on the top of the third linking rod 13, and encoders 16 which are fixed mounted on the first joint 14 and the second joint 15.

The side wall of the third linking rod 13 is provided with a clutch button 19; the clutch button 19 is arranged directly below the clamping unit 18 and is wirely connected with operative system; when the hand of a surgeon reaches an operative movement limit, the clutch button 19 is pressed to allow a disengagement of the hand held portion 10 and the operative end portion 40, after the hand held portion 10 is adjusted to a new operative position, the clutch button 19 is then released to allow reengagement and the surgeon can continue with the operation.

As shown in Fig. 4, the clamping unit 18 which comprising a base panel 184, the base panel 184 is provided with a pair of symmetrically opposing planar linkage mechanisms, each of the linkage mechanisms which comprising a mover lever 185 and a lower lever 186, one end of the mover lever 185 is rotablely mounted on the base panel 184, and the other end of the mover lever 185 is hinged to one end of the lower lever 186, and the other end of the lower lever 186 is rotablely mounted on a protruding end of a potentiometer 182, the potentiometer 182 is fix mounted on the base panel 184, a spring 183 is provided in between the mover levers of the pair linkage mechanisms. The two ends of the spring 183 are abutting against the two mover levers 185, and the side wall of the mover lever 185 is provided with a clamping piece 181.

The third linking rod 13 is further provided with a bracelet 50, and the bracelet 50 is arranged directly below the clutch button 19. Wherein, the bracelet 50 and the clutch button 19 are arranged on a same plane of the third linking rod. The bracelet 50 which comprising a ring portion 52 and connecting ends 51 which are fix arranged at two ends of the ring portion 52. The connecting ends 51 are fix mounted on the third linking rod 13. Wherein, the ring portion 52 is made of elastic material and the connecting ends 51 are made of hard material. The ring portion 52 is in a C shape for receiving the three holding fingers of the surgeon, and as the ring portion 52 is made of elastic material, it is fitted for use of different surgeons.

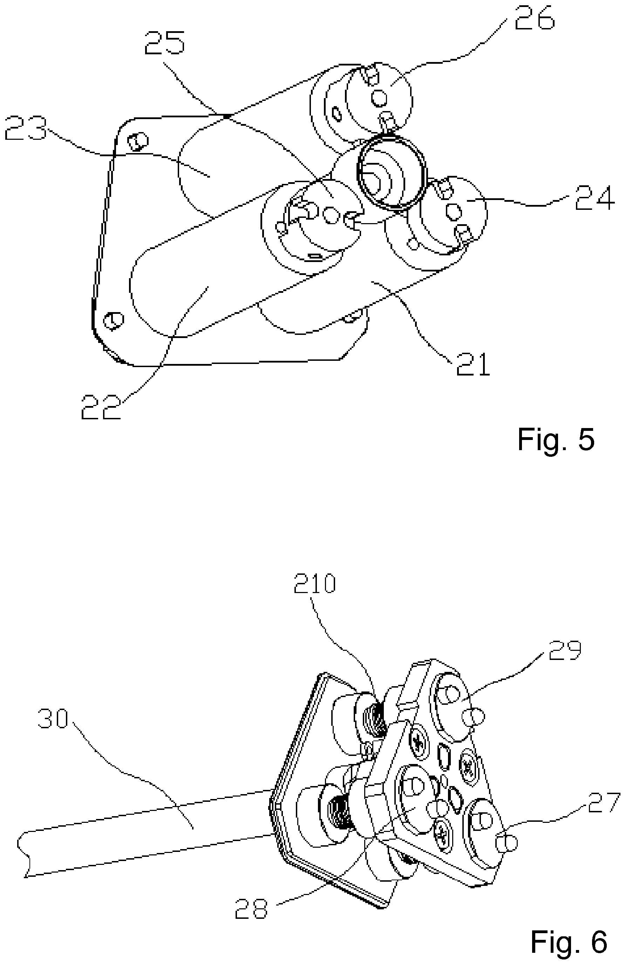

As shown in Fig. 5 and Fig. 6, the power portion 20 which comprising an outer cover (not shown), the outer cover is provided with a yaw motor 21, a pitch motor 22 and a clamping motor 23 within. An output shaft end of the yaw motor 21 is connected with an yaw motor active dial 24, an output shaft end of the pitch motor 22 is connected with a pitch motor active dial 26, and an output shaft end of the clamping motor 23 is connected with a clamping motor active dial 26; the yaw motor active dial 24 is connected with a yaw motor passive dial 27, the pitch motor active dial 25 is connected with a pitch motor passive dial 28, and the clamping motor active dial 26 is connected with a clamping motor passive dial 29. The yaw motor passive dial 27, the pitch motor passive dial 28 and the clamping motor passive dial 29 are respectively connected with a metal wire turning apparatus 210.

One end of the thin linking rod 30 is fix arranged within the power portion 20 and the other end of the thin linking rod 30 extends out the power portion 20. Wherein, the thin linking rod 30 is a hollow structure, and several fixing slots are provided within the hollow structure. The metal wire turning apparatus 210 are fix arranged within the thin linking rod 30 through the fixing slots. The operative end portion 40 connects with the thin linking rod 30 at one end that is away from the power portion 20, and the operative end portion 40 also connects with the metal wire turning apparatus 210.

Wherein, the hand held portion 10 and the power portion 20 are both wire connected with the operative system. The operation of the hand held portion 10 drives the encoder 16 on the joints to operative, the encoder 16 collects the operative information of the joints and then feeds back to the operative system, after the power portion 20 receives signals from the operative system, the power portion 20 drives the operative end portion 40 to complete a yaw, pitch and clamping motion.

When operating, a surgeon would hold onto the third linking rod 13, the thumb and the index finger of the surgeon would press and hold on the clamping piece 181 on the two mover levers 185. The spring action of the spring 183 allows the clamping piece 181 to open automatically, and the pressing force from the finger would cancel the force from the spring 183 to complete a closing action. During the clamping process the electrical resistance of the potentiometer 182 changes, causing the voltage of the potentiometer 182 to change. This change in voltage triggers an operative signal to the operative end portion 40 to perform a clamping action. The encoder 16 on the first joint 14 and the second joint 15 collects information from the second linking rod 12 and the third linking rod 13 and feeds back to the operative system, and the power portion 20 controls the operative end portion 40 to perform corresponding yaw and pitch motion. In addition, the turning of the arm of the surgeon can be reflected as an axis rotation of the operative end portion 40.

## Claims

1. A minimally invasive surgical module for minimally invasive surgery which comprising a hand held portion (10), a power portion (20) which is fix connected with the hand held portion (10), a thin linking rod (30) which is fix connected with the power portion (20), an operative end portion (40) which is connected with the thin linking rod (30), and an operative system for controlling the operation of the power portion (20); wherein, the hand held portion (10) which comprising a first linking rod (11) that one end of the linking rod (11) is fix connected with the power portion (20), a second linking rod (12) which through a first joint (14), is rotablely connected with the first linking rod (11), a third linking rod (13) which through a second joint (15), is rotablely connected with the second linking rod (12), a clamping unit (18) which through a clamping cap (17), is fixed mounted on the top of the third linking rod (13), and encoders (16) which are fixed mounted on the first joint (14) and the second joint (15); wherein, the clamping unit (18) which comprising a base panel (184), the base panel (184) is provided with a pair of symmetrically opposing planar linkage mechanism and springs (183), each of the linkage mechanisms which comprising a mover lever (185) and a lower lever (186), one end of the mover lever (185) is rotablely mounted on the base panel (184), and the other end of the mover lever (185) is hinged to one end of the lower lever (186), and the other end of the lower lever (186) is rotablely mounted on a protruding end of a potentiometer (182), the potentiometer (182) is fix mounted on the base panel (184), a spring (183) is provided in between the mover levers of the pair linkage mechanisms; wherein, the two ends of the spring (183) are abutting against the two mover levers (185), and the side wall of the mover lever (185) is provided with a clamping piece (181); wherein, the hand held portion (10) and the power portion (20) are both wire connected with the operative system; wherein, the operation of the hand held portion (10) drives the encoder (16) on the joints to operative, the encoder (16) collects the operative information of the joints and then feeds back to the operative system, after the power portion (20) receives signals from the operative system, the power portion (20) drives the operative end portion (40) to complete a yaw, pitch and clamping motion.

2. The minimally invasive surgical module for minimally invasive surgery as claimed in claim 1 wherein, the power portion (20) which comprising an outer cover, the outer cover is provided with a yaw motor (21), a pitch motor (22) and a clamping motor (23) within; wherein, an output shaft end of the yaw motor (21) is connected with an yaw motor active dial (24), an output shaft end of the pitch motor (22) is connected with a pitch motor active dial (26), and an output shaft end of the clamping motor (23) is connected with a clamping motor active dial (26); the yaw motor active dial (24) is connected with a yaw motor passive dial (27), the pitch motor active dial (25) is connected with a pitch motor passive dial (28), and the clamping motor active dial (26) is connected with a clamping motor passive dial (29); and wherein, the yaw motor passive dial (27), the pitch motor passive dial (28) and the clamping motor passive dial (29) are respectively connected with a metal wire turning apparatus (210).

3. The minimally invasive surgical module for minimally invasive surgery as claimed in claim 2 wherein, one end of the thin linking rod (30)is fix arranged within the power portion (20) and the other end of the thin linking rod (30) extends out the power portion (20); wherein, the thin linking rod (30) is a hollow structure, and several fixing slots are provided within the hollow structure; and wherein, the metal wire turning apparatus (210) are fix arranged within the thin linking rod (30) through the fixing slots.

4. The minimally invasive surgical module for minimally invasive surgery as claimed in claim 3 wherein, the operative end portion (40) connects with the thin linking rod (30) at one end that is away from the power portion (20), and the operative end portion (40) also connects with the metal wire turning apparatus (210).

5. The minimally invasive surgical module for minimally invasive surgery as claimed in one of the preceding claims wherein a side wall of the third linking rod (13) is provided with a clutch button (19); and wherein, the clutch button (19) is arranged directly below the clamping unit (18) and is wirely connected with operative system.

6. The minimally invasive surgical module for minimally invasive surgery as claimed in claim (5) wherein, the third linking rod (13) is further provided with a bracelet (50), and the bracelet (50) is arranged directly below the clutch button (19); and wherein, the bracelet (50) and the clutch button (19) are arranged on a same plane of the third linking rod.

7. The minimally invasive surgical module for minimally invasive surgery as claimed in claim 6 wherein, the bracelet (50) which comprising a ring portion (52) and connecting ends (51) which are fix arranged at two ends of the ring portion (52); wherein, the connecting ends (51) are fix mounted on the third linking rod (13); and wherein, the ring portion (52) is made of elastic material and the connecting ends (51) are made of hard material.
